# DEMANDE DE BREVET EUROPEEN

(11) **EP 1 075 845 A1**
(43) Date de publication de la demande: **14.02.2001**
(21) Numéro de dépôt: 00402059.0
(22) Date de dépôt: 19.07.2000
(51) Int. Cl.: A61M 1/36, A61M 5/165

(54) **Ligne de transfusion avec moyens de filtration d'une substance virucide intégrés**

(30) Priorité: 09.08.1999 FR 9910323
(71) Demandeur: MACO PHARMA, F-59200 Tourcoing (Nord) (FR)
(72) Inventeur: Demay, Sylvie, 59200 Tourcoing (FR); Verpoort, Thierry, 59420 Mouvaux (FR); Goudaliez, Francis, 59155 Faches Thumesnil (FR)
(74) Mandataire: Keib, Gérard

(57) **Abrégé**

L'invention a pour objet une ligne de transfusion (1) d'un fluide biologique chez un receveur, du type comprenant au moins une tubulure (2) ayant une partie d'entrée (2a) pourvue à son extrémité amont (22) de moyens (3) de mise en communication ou de moyens de connexion avec des moyens de mise en communication de la ligne de transfusion (1) avec le contenu d'une poche contenant le fluide biologique additionné d'une substance virucide, et une partie de sortie (2b) munie à son extrémité aval (19) de moyens de transfusion (4) ou de moyens de connexion avec des moyens de transfusion, des moyens de filtration (14) apte à éliminer la substance virucide présente dans le fluide biologique sont intégrés entre la partie d'entrée (2a) et la partie de sortie (2b) de la tubulure (2).

## Description

L'invention se rapporte à une ligne de transfusion d'un fluide biologique chez un receveur.

La transfusion de fluide biologique à un patient, notamment du plasma, à des intervalles de temps réguliers, est une opération courante en milieu hospitalier. Généralement, ces transfusions sont réalisées auprès du lit du malade.

Avant d'être transfusé au patient, le fluide biologique peut avoir subit un traitement d'inactivation virale de sorte à inactiver d'éventuels virus qui contaminent le fluide biologique.

Une technique classique d'inactivation virale du plasma utilise une substance virucide, par exemple le bleu de méthylène, qui est additionnée au fluide biologique.

Le principe de cette technique repose sur des réactions photochimiques entre la substance virucide et l'ADN ou l'ARN viral éventuellement présent dans le fluide biologique. L'exposition à la lumière de la substance virucide entraîne une réaction photochimique qui transmet de l'énergie aux molécules d'ADN et d'ARN de sorte que le virus est inactivé.

Lors de ces réactions photochimiques, la substance virucide n'est pas éliminée de sorte qu'elle reste dans le fluide biologique après l'exposition à la lumière.

Après la mise en oeuvre de cette technique d'inactivation virale, la substance virucide, en quantité très faible, peut être laissée dans le fluide biologique et ainsi être transfusé au patient en même temps que celui-ci.

Cependant, des études récentes semblent montrer la possible toxicité de certaines substances virucides utilisées, et notamment du bleu de méthylène, lorsqu'elles sont injectées au patient.

De sorte que de nombreux pays demandent l'élimination systématique des substances virucides préalablement à l'injection du fluide biologique au patient.

On connaît déjà, par exemple dans le document WO-A-95/00631, des filtres capables d'éliminer des substances virucides qui sont intégrés à un dispositif d'inactivation virale.

Les documents US-5 858 641 et EP-0 933 090 illustrent également cette technologie en proposant des systèmes dans lesquels le fluide est inactivé, filtré puis recueilli dans une poche.

Par ailleurs, le document US-4 915 683 décrit un dispositif d'inactivation virale dans lequel le fluide est d'abord inactivé puis transfusé au patient.

De tels dispositifs présentent des inconvénients.

En particulier, l'inactivation virale du fluide biologique est rendue plus compliquée du fait de l'exécution de l'étape supplémentaire de filtration.

Cet inconvénient est très pénalisant dans le cadre de l'exigence d'une élimination systématique de la substance virucide additionnée au fluide biologique.

L'invention vise donc à remédier à ces inconvénients, en proposant d'éliminer la substance virucide au lit du malade et non lors de l'inactivation virale, cette opération s'effectuant dans un dispositif différent de celui servant à l'inactivation virale et en temps masqué.

A cet effet, l'invention a pour objet une ligne de transfusion d'un fluide biologique chez un receveur, du type comprenant au moins une tubulure ayant une partie d'entrée pourvue à son extrémité amont de moyens de mise en communication ou de moyens de connexion avec des moyens de mise en communication de la ligne de transfusion avec le contenu d'une poche contenant le fluide biologique additionné d'une substance virucide, et une partie de sortie munie à son extrémité aval de moyens de transfusion ou de moyens de connexion avec des moyens de transfusion, des moyens de filtration apte à éliminer la substance virucide présente dans le fluide biologique étant intégrés entre la partie d'entrée et la partie de sortie de la tubulure.

Les moyens de filtration sont intégrés à demeure à la ligne de transfusion de sorte à former avec elle un ensemble unitaire prémonté comme tel.

Dans une première variante, les moyens de filtration comprennent un filtre rigide.

Dans une deuxième variante, les moyens de filtration comprennent un filtre souple.

Dans un premier mode de réalisation, les moyens de filtration comprennent un milieu filtrant réalisé à partir d'un matériau non tissé spécifiquement traité pour éliminer les substances virucides. Le matériau non tissé peut se présenter sous la forme de plusieurs couches ou être disposé et tassé dans les moyens de filtration.

Dans un deuxième mode de réalisation, les moyens de filtration comprennent un milieu filtrant réalisé sous la forme d'une poudre apte à retenir les substances virucides, notamment par affinité.

La ligne de transfusion comprend en amont des moyens de filtration des moyens de préfiltration destinés à recueillir les micro agrégats éventuellement présents dans le fluide biologique.

D'autres objets et avantages de l'invention apparaîtront au cours de la description qui suit en référence au dessin annexé.

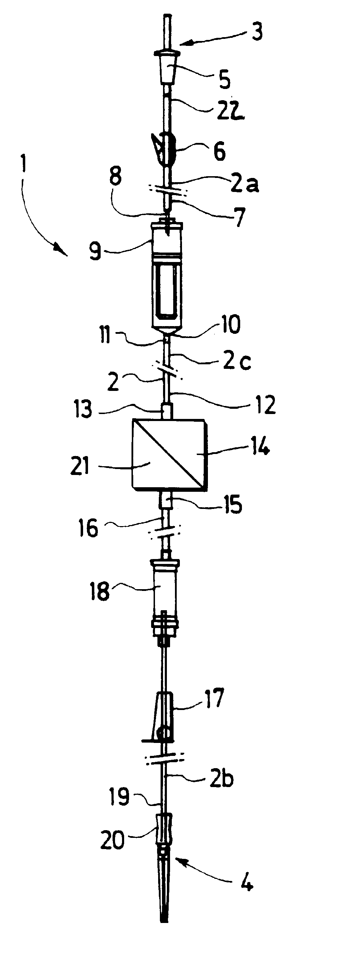

La figure 1 représente de façon schématique un mode de réalisation de la ligne de transfusion de l'invention.

Une ligne de transfusion 1 comprend typiquement une tubulure 2 ayant une partie d'entrée 2a munie de moyens 3 de mise en communication de la ligne de transfusion 1 avec le contenu d'une poche (non représentée) contenant un fluide biologique et une partie de sortie 2b munie de moyens de transfusion 4. Les moyens 3 de mise en communication sont en communication fluidique avec les moyens de transfusion 4 par l'intermédiaire de la tubulure 2. La tubulure 2 de la ligne de transfusion 1 est par exemple souple, sécable et soudable.

La ligne de transfusion 1 est destinée d'une part à être connectée à la poche contenant le fluide biologique à transfuser par l'intermédiaire des moyens 3 de mise en communication et d'autre part au patient auquel le fluide biologique doit être administré par l'intermédiaire des moyens de transfusion 4, par exemple comprenant un luer 20 et une aiguille de perfusion.

La ligne de transfusion 1 permet, par l'intermédiaire de la tubulure 2, de délivrer au patient le fluide biologique contenu dans la poche.

Le fluide biologique est par exemple un produit sanguin et plus particulièrement du plasma sanguin.

Préalablement à la connexion de la poche sur la ligne de transfusion 1, le fluide biologique a subit un traitement d'inactivation virale au moyen d'une substance virucide, par exemple colorante à base de bleu de méthylène, qui est introduit dans la poche.

Ce traitement, généralement mise en oeuvre au centre de transfusion sanguine, ne sera pas plus décrit ici.

Après la réalisation de l'inactivation virale du fluide biologique, la poche contient donc le fluide biologique exempt de virus qui doit être injecté au patient et la substance virucide qui a servi à inactiver les virus.

Au lit du patient, la poche est alors connectée à la ligne 1 par l'intermédiaire des moyens 3 de mise en communication.

Les moyens 3 de mise en communication sont destinés à permettre le passage du fluide biologique additionné de la substance virucide qui sont contenus dans la poche, dans la partie d'entrée 2a de la tubulure 2 de la ligne de transfusion 1.

A cet effet, la partie d'entrée 2a de la tubulure 2 est munie à son extrémité amont 22 d'un dispositif de perforation 5 qui forme les moyens 3 de mise en communication.

Dans la description, les termes "amont" et "aval" sont définis par rapport au sens de circulation du fluide biologique à transfuser, c'est à dire du dispositif de perforation 5 vers les moyens de transfusion 4.

La partie d'entrée 2a de la tubulure 2 peut être munie d'un système d'ouverture/fermeture de la communication tel qu'un clamp 6 permettant de contrôler le passage du fluide biologique dans la tubulure 2.

La partie d'entrée 2a de la tubulure 2 communique par son extrémité aval 7, c'est à dire celle opposée au dispositif de perforation 5, avec l'orifice d'entrée 8 d'une chambre de préfiltration 9 apte à recueil des micro agrégats éventuellement présents dans le fluide biologique. Cette chambre de préfiltration 9 est classique en elle-même et ne sera donc pas décrite plus avant.

La chambre 9 comporte un orifice de sortie 10 communiquant avec une extrémité amont 11 d'une deuxième partie 2c de la tubulure 2.

L'extrémité aval 12 de la partie 2c de la tubulure 2 communique avec un orifice d'entrée 13 d'un filtre 14 apte à éliminer la substance virucide présente dans le fluide biologique dont la structure sera décrite plus en détail dans ce qui suit.

Le filtre 14 comprend un orifice de sortie 15 par lequel le fluide biologique exempt de substance virucide est délivré.

Une première extrémité 16, amont, de la partie de sortie 2b de la tubulure 2 est connectée de façon étanche à l'orifice de sortie 15 du filtre 14.

La partie de sortie 2b de la tubulure 2 comprend un dispositif compte gouttes 18 et une roulette de réglage de débit 17, permettant de réguler et de visualiser le débit du fluide biologique transfusé au patient.

La partie de sortie 2b de la tubulure 2 comprend à son extrémité aval 19, c'est à dire celle opposée au filtre 14, des moyens de transfusion 4, par exemple formés d'un luer 20 destiné à être connecté à une aiguille de perfusion ou a un cathéter introduit dans une veine du patient de sorte à lui transfuser le fluide biologique exempt de substance virucide. En variante, les moyens de transfusion 4 peuvent être remplacés par des moyens aptes à mettre la ligne de transfusion en communication avec des moyens de transfusion extérieurs à la ligne de transfusion.

La ligne de transfusion 1 qui vient d'être décrite constitue un ensemble élémentaire qui peut être incorporé dans un système plus complexe qui comprend par exemple d'autres poches, des tubulures, des clamps ou des filtres (par exemple à déleucocyter), lesquelles éléments ne seront pas décrit plus avant.

On décrit maintenant plus en détail le filtre 14 apte à éliminer la substance virucide présente dans le fluide biologique.

Le filtre 14 comprend un compartiment de filtration 21 dans lequel un milieu filtrant est disposé. Les orifices d'entrée 13 et de sortie 15 du filtre 14 sont disposés sur le compartiment 21, par exemple de part et d'autre de celui-ci, de sorte que le chemin d'écoulement du fluide biologique s'étende de l'un à l'autre via le milieu filtrant.

Le fluide biologique additionné de la substance virucide pénètre dans le filtre 14 par l'orifice d'entrée 13, traverse le milieu filtrant de sorte à ce que la substance virucide soit absorbée ou adsorbée par le milieu filtrant, puis le fluide biologique exempt de substance virucide sort du filtre 14 par l'orifice de sortie 15 pour être délivré au patient.

Selon une première variante d'exécution, le filtre 14 est rigide. Par exemple, un plastique rigide, tel que du polycarbonate, est utilisé pour réaliser l'enveloppe extérieure du compartiment de filtration 21.

Selon une deuxième variante d'exécution le compartiment de filtration 21 est réalisé par une poche en matériau plastique souple.

Dans un premier mode de réalisation, le milieu filtrant est réalisé à partir d'un matériau non tissé spécifiquement traité pour éliminer les substances virucides.

Le matériau non tissé est par exemple formé de polymères et/ou copolymères à base de polypropylène, de polyester, de polyamide, de polyéthylène haute ou basse densité, de polyuréthane.

Un exemple particulier de polymère utilisable est le fluorure de polyvinylidène

Le matériau non tissé peut avoir subit un traitement physique ou chimique, notamment plasma, de sorte à créer, en surface ou en profondeur, des sites d'absorption ou d'adsorption des substances virucides.

Dans une première variante, le matériau non tissé se présente sous la forme de plusieurs couches qui sont disposées dans le compartiment de filtration 21. Ces couches peuvent être identiques et/ou différentes, notamment par leur composition et/ou la nature du traitement qu'elles ont subit.

Dans une seconde variante, le matériau non tissé est disposé puis tassé dans le compartiment de filtration 21.

Dans un deuxième mode de réalisation, le milieu filtrant est réalisé sous la forme d'une poudre apte à retenir les substances virucides.

Le milieu filtrant exerce alors une fonction chromatographique par affinité en absorbant sélectivement la substance virucide présente dans le fluide biologique.

La poudre est par exemple formée de charbon actif ayant la capacité d'absorber les substances virucides. En variante, la poudre peut être disposée entre deux couches de matériau non tissé.

On décrit ci-après sommairement le fonctionnement de la ligne de transfusion 1 de l'invention.

Après la mise en oeuvre d'une technique d'inactivation virale du fluide biologique, la poche contenant le fluide biologique à transfuser additionné de la substance virucide à éliminer est connectée à la partie d'entrée 2a de la tubulure 2 par l'intermédiaire du dispositif de perforation 5.

Le clamp 6 est alors ouvert pour autoriser le passage du fluide biologique contenu dans la poche reliée au dispositif de perforation 5.

Le fluide biologique passe dans la tubulure 2 et les micro agrégats éventuellement présents dans le fluide biologique sont retenus par la chambre de préfiltration 9.

Le fluide biologique additionné de la substance virucide arrive ensuite dans le compartiment de filtration 21 du filtre 14 où il est débarrassé de la majeure partie de la substance virucide.

Le fluide biologique exempt de substance virucide sort du compartiment de filtration 21 par l'orifice de sortie 15 et passe, par l'intermédiaire de la partie de sortie 2b de la tubulure 2 jusqu'au luer 20 connecté à une aiguille ou à un cathéter qui a été préalablement introduit dans une veine du patient de sorte que le fluide exempt de substance virucide lui soit administré.

## Revendications

1. Ligne de transfusion (1) d'un fluide biologique chez un receveur, du type comprenant au moins une tubulure (2) ayant une partie d'entrée (2a) pourvue à son extrémité amont (22) de moyens (3) de mise en communication ou de moyens de connexion avec des moyens de mise en communication de la ligne de transfusion (1) avec le contenu d'une poche contenant le fluide biologique additionné d'une substance virucide, et une partie de sortie (2b) munie à son extrémité aval (19) de moyens de transfusion (4) ou de moyens de connexion avec des moyens de transfusion, caractérisée en ce que des moyens de filtration (14) apte à éliminer la substance virucide présente dans le fluide biologique sont intégrés entre la partie d'entrée (2a) et la partie de sortie (2b) de la tubulure (2).

2. Ligne de transfusion selon la revendication 1, caractérisée en ce que les moyens de filtration (14) sont intégrés à demeure à la ligne de transfusion (1) de sorte à former avec elle un ensemble unitaire prémonté comme tel.

3. Ligne de transfusion selon la revendication 1 ou 2, caractérisée en ce que les moyens de filtration (14) comprennent un filtre rigide.

4. Ligne de transfusion selon la revendication 1 ou 2, caractérisée en ce que les moyens de filtration (14) comprennent un filtre souple.

5. Ligne de transfusion selon l'une quelconque des revendications 1 à 4, caractérisée en ce que les moyens de filtration (14) comprennent un milieu filtrant réalisé à partir d'un matériau non tissé spécifiquement traité pour éliminer les substances virucides.

6. Ligne de transfusion selon la revendication 5, caractérisée en ce que le matériau non tissé se présente sous la forme de plusieurs couches.

7. Ligne de transfusion selon la revendication 5, caractérisée en ce que le matériau non tissé est disposé et tassé dans les moyens de filtration (14).

8. Ligne de transfusion selon l'une quelconque des revendications 1 à 4, caractérisée en ce que les moyens de filtration (14) comprennent un milieu filtrant réalisé sous la forme d'une poudre apte à retenir les substances virucides, notamment par affinité.

9. Ligne de transfusion selon l'une quelconque des revendications 1 à 8, caractérisée en ce qu'elle comprend en amont des moyens de filtration (14) des moyens de préfiltration (9) destinés à recueillir les micro-agrégats éventuellement présents dans le fluide biologique.
